# EUROPEAN PATENT SPECIFICATION

(11) **EP 3 431 131 B1**
(45) Date of publication and mention of the grant of the patent: **11.11.2020**
(21) Application number: 16829395.9
(22) Date of filing: 15.03.2016
(51) Int. Cl.: A61M 25/10, A61M 25/01

(54) **BALLOON CATHETER**
BALLONKATHETER
CATHÉTER À BALLONNET

(43) Date of publication of application: 23.01.2019
(73) Proprietor: ASAHI INTECC CO., LTD., Seto-shi, Aichi 489-0071 (JP)
(72) Inventor: TSUKAMOTO, Toshihiko, Seto-shi Aichi, 489-0071 (JP); MIKI, Akihiro, Seto-shi, Aichi 489-0071 (JP)
(74) Representative: Winter, Brandl, Fürniss, Hübner, Röss, Kaiser, Polte - Partnerschaft mbB
(86) International application number: PCT/JP2016/058198
(87) International publication number: WO 2017/158735

(56) References cited:
- EP-A1- 1 076 577
- JP-A- H01 192 369
- JP-A- H11 128 350
- JP-A- 2001 238 957
- JP-A- 2015 156 880
- US-A- 5 512 051
- US-A1- 2002 082 549
- US-A1- 2014 172 003

## Description

### Field

The present invention relates to a balloon catheter.

### Background

Conventionally, there are known a medical treatment balloon catheter that expands a constricted portion formed in a blood vessel or a digestive organ to recover a flow of blood or a digestion liquid, and a fixing balloon catheter that easily facilitates operation of inserted medical equipment such as a guide wire by expanding a balloon to a blood vessel wall or a digestive organ wall and fixing the balloon. Such balloon catheters mainly include a balloon that is an expansion body, an outer shaft fixed to a proximal end of the balloon, and an inner shaft inserted in the balloon and the outer shaft. Generally, the inner shaft is used to allow a guide wire to pass therethrough, and an expansion lumen provided between the outer shaft and the inner shaft is used to allow liquid for expanding the balloon (contrast medium or physiological saline) to flow.

The balloon catheter has a problem that with low fixing strength between the proximal end of the balloon and the outer shaft, the proximal end of the balloon is removed from a distal end of the outer shaft when the balloon is expanded to expand a constricted portion or to fix the balloon to a blood vessel wall or a digestive organ wall.

As a method of solving this problem, there are known a balloon catheter improved in fixing strength between the balloon and the outer shaft by coating, from the outer periphery, the proximal end of the balloon and the distal end of the outer shaft by a heat shrinkable tube that is a separate member (see JP 2009-056297 A, for example), and a balloon catheter improved in fixing strength between the balloon and the distal end of the outer shaft by providing a mutually diagonally-cut overlapped part between the proximal end of the balloon and the distal end of the outer shaft (see JP 5237572 B2, for example).

However, the balloon catheter described in JP 2009-056297 A is coated, from the outer periphery, by a heat shrinkable tube that is a separate member. Thus, an outer diameter of the fixing part between the balloon and the outer shaft becomes large, thereby causing a problem that the passing performance of the balloon catheter is deteriorated. Moreover, in the balloon catheter described in JP 5237572 B2, an outer diameter of the fixing part between the balloon and the outer shaft is not large. However, the expansion of the fixing area between the balloon and the outer shaft has a limit, which means improvement of the fixing strength between the balloon and the outer shaft has a limit. Especially when high pressure is imposed on the balloon, there is a risk that the proximal end of the balloon is removed from the distal end of the outer shaft.

EP 1076577 A1 discloses a balloon for a medical device, the balloon comprising a plurality of balloon segments. Specifically, the balloon have a distal segment, a body segment and a proximal segment.

US 2014172003 A1 discloses a catheter assembly comprising a tube member having a first tube end and a second tube end with a lumen extending therebetween and a balloon. The balloon is in fluid communication with the lumen. The balloon comprises a first balloon end threadably coupled to the tube member. By this thread coupling, the process of coupling the balloon to the tube member is simplified.

US 5512051 A discloses a catheter having a balloon and a shaft. The balloon includes two layers, an inner layer and an outer layer. The proximal end of the balloon is fixed to the shaft by a sleeve cover and adhesive. The distal end of the balloon is fixed to a wire by a sleeve cover.

US 2002082549 A1 discloses a balloon catheter including a balloon, a shaft and an inner tubular member extending inside the shaft. The proximal end of the balloon is fusion-bonded to the distal end of the shaft. The distal end of the balloon is fusion-bonded to the inner tubular member. The inner tubular member includes two sections, a distal portion and a distal tip extending from the distal end of the distal portion. The distal end of the balloon is fusion-bonded to the inner tubular member at the interface between the distal portion and the distal tip, of the inner tubular member.

### Summary

### Technical Problem

Starting from JP 5237572 B2, the present invention aims at providing a balloon catheter improved in fixing strength between a balloon and an outer shaft without increasing an outer diameter of a fixing part between the balloon and the outer shaft.

### Solution to Problem

This object is solved by a balloon catheter in accordance with Claim 1. Preferred embodiments thereof are subject matter of dependent claims.

According to the invention, a balloon catheter comprises a balloon, and an outer shaft fixed to a proximal end of the balloon, in which each of the proximal end of the balloon and a distal end of the outer shaft has an inner projection extending in an axis direction, an outer projection extending in the axis direction, and a gap formed between the inner projection and the outer projection. Furthermore, either of i) or ii) is met. i) The inner projection of the balloon is disposed in the gap of the outer shaft and the outer projection of the outer shaft is disposed in the gap of the balloon. ii) The outer projection of the balloon is disposed in the gap of the outer shaft and the inner projection of the outer shaft is disposed in the gap of the balloon.

In a preferred embodiment, a bulging portion is provided at the inner projection of the balloon or the outer projection of the balloon that is disposed in the gap of the outer shaft.

In a further preferred embodiment, a bulging portion is provided at the inner projection of the outer shaft or the outer projection of the outer shaft that is disposed in the gap of the balloon.

### Advantageous Effects of Invention

In the balloon catheter according to the invention, an inner projection extending in an axis direction, an outer projection extending in the axis direction, and a gap formed between the inner projection and the outer projection are provided at each of the proximal end of the balloon and a distal end of the outer shaft. Furthermore, i) the inner projection of the balloon is disposed in the gap of the outer shaft and the outer projection of the outer shaft is disposed in the gap of the balloon, or ii) the outer projection of the balloon is disposed in the gap of the outer shaft and the inner projection of the outer shaft is disposed in the gap of the balloon. Thus, a fixing area between the balloon and the outer shaft can be expanded easily. Therefore, it is possible to improve the fixing strength between the balloon and the outer shaft without increasing an outer diameter at the fixing part between the balloon and the outer shaft. Moreover, the proximal end of the balloon and the distal end of the outer shaft are engaged with each other. Thus, it is possible to reduce a risk that the proximal end of the balloon is removed from the distal end of the outer shaft even when a high pressure is imposed on the balloon.

A preferred embodiment of a bulging portion is provided at the inner projection of the balloon or the outer projection of the balloon that is disposed in the gap of the outer shaft. A fixing area between the balloon and the outer shaft can be further expanded, and the anchoring effect of the bulging portion relative to the gap of the outer shaft can further improve the fixing strength between the balloon and the outer shaft.

Further, if a bulging portion is provided at the inner projection of the outer shaft or the outer projection of the outer shaft that is disposed in the gap of the balloon, a fixing area between the balloon and the outer shaft can be further expanded, and the anchoring effect of the bulging portion relative to the gap of the balloon can further improve the fixing strength between the balloon and the outer shaft.

### Brief Description of Drawings

FIG. 1 is a diagram illustrating an entire view of a balloon catheter according to an embodiment.
FIG. 2 is an enlarged view of an A part of FIG. 1.
FIG. 3 is a diagram illustrating a state where a balloon and an outer shaft that are illustrated in FIG. 2 are separated from each other.
FIG. 4 is a diagram illustrating a part of a balloon catheter according to a second embodiment, and is a modification of FIG.2.
FIG. 5 is a diagram illustrating a part of a balloon catheter according to a third embodiment. FIG. 5A is a modification of FIG. 2, and FIG. 5B is a modification of FIG. 4.
FIG. 6 is a diagram illustrating a part of a balloon catheter according to a fourth embodiment. FIG. 6A is a modification of FIG. 2, and FIG. 6B is a modification of FIG. 4.
FIG. 7 is a diagram illustrating a part of a balloon catheter according to a fifth embodiment. FIG. 7A is a modification of FIG. 6A, and FIG. 7B is a modification of FIG. 6B.
FIG. 8 is a diagram illustrating a part of a balloon catheter according to a sixth embodiment. FIG. 8A is a modification of FIG. 7A, and FIG. 8B is a modification of FIG. 7B.
FIG. 9 is a modification of FIG. 3.
FIG. 10 is a diagram illustrating an entire view of a balloon catheter according to a seventh embodiment.

### Description of Embodiments

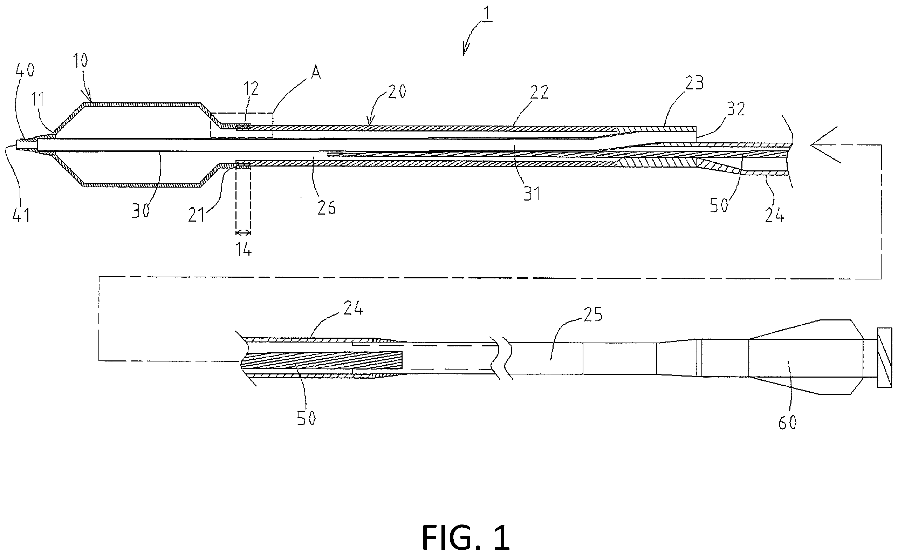

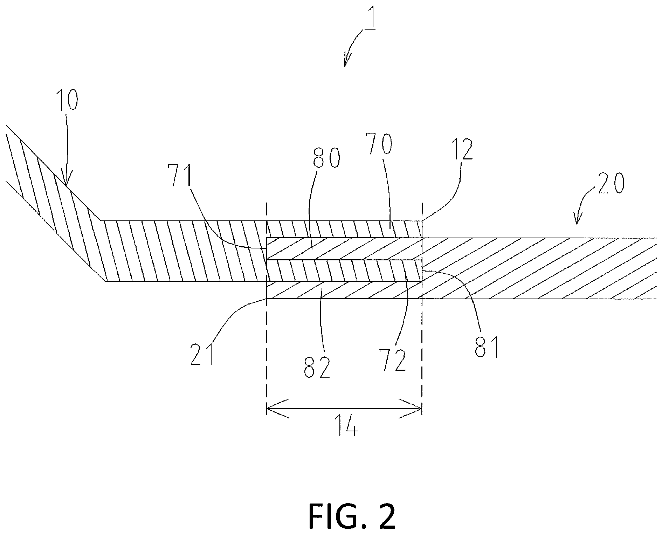

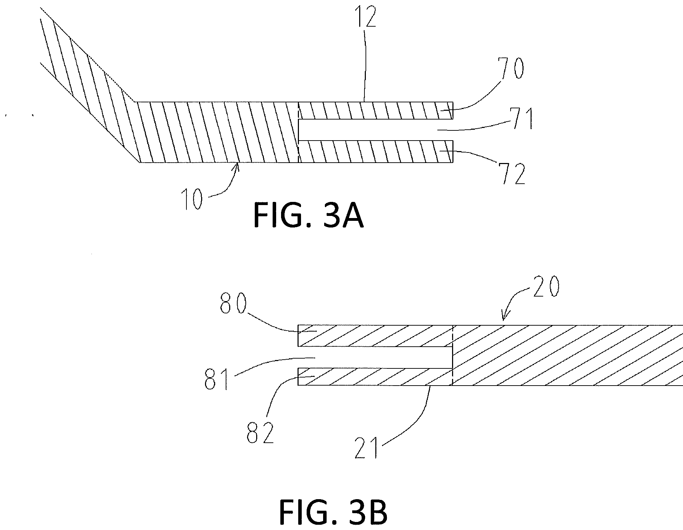

A balloon catheter 1 according to an embodiment will be described with reference to FIG. 1 to FIG. 3. In FIG. 1, the left side of the drawings is a distal end side to be inserted in a body (far side), while the right side is a proximal end side to be operated by a technician such as a physician (near side, rear end side). FIG. 2 is an enlarged view of an A part of FIG. 1. FIG. 3 is a diagram illustrating, for facilitating understanding, a state where a balloon and an outer shaft that are illustrated in FIG. 2 are separated from each other.

The balloon catheter 1 is for example, a medical treatment balloon catheter used to expand a constricted portion for treatment. As illustrated in FIG. 1, the balloon catheter 1 mainly includes a balloon 10, an outer shaft 20, an inner shaft 30, a distal end tip 40, a reinforcing body 50, and a connector 60.

The balloon 10 expanding a constricted portion is formed of a resin member, and includes a distal end attachment part 11 at a distal end of the balloon 10, and a proximal end attachment part 12 at a proximal end of the balloon 10. The distal end attachment part 11 is fixed to a distal end of the inner shaft 30 through the distal end tip 40, and the proximal end attachment part 12 is fixed to a distal end 21 of the outer shaft 20. However, the embodiment is not limited thereto. For example, the distal end attachment part 11 may be held between the inner shaft 30 and the distal end tip 40 so that the distal end attachment part 11 of the balloon 10 is directly fixed to the inner shaft 30.

The outer shaft 20 is a tubular member forming an expanded lumen 26 for supplying liquid such as a contrast medium or physiological saline to expand the balloon 10. The outer shaft 20 includes a distal end outer shaft part 22, a guide wire port part 23, an intermediate outer shaft part 24, and a proximal end outer shaft part 25 in this order from the distal end side. The distal end outer shaft part 22 and the intermediate outer shaft part 24 are tubes formed of resin of polyamide, polyamide elastomer, polyolefin, polyester, and polyester elastomer. The guide wire port part 23 is a part where the distal end outer shaft part 22, the intermediate outer shaft part 24, and the inner shaft 30 are fixed to one another.

The inner shaft 30 is inserted in the distal end outer shaft part 22, and the above-described expanded lumen 26 is formed between the distal end outer shaft part 22 and the inner shaft 30.

The proximal end outer shaft part 25 is a metallic tubular member of a so-called hypo tube. The distal end of the proximal end outer shaft part 25 is inserted and fixed to the proximal end of the intermediate outer shaft part 24. A connector 60 is attached to the proximal end of the proximal end outer shaft part 25. When liquid such as a contrast medium or physiological saline for expanding the balloon 10 is supplied from an indeflator (not illustrated) that can be attached to the connector 60, the liquid passes through the expanded lumen 26 and expands the balloon 10. Note that the material of the proximal end outer shaft part 25 is not particularly limited, and stainless steel (SUS302, SUS304) or a superelastic alloy such as a Ni-Ti alloy can be used.

The inner shaft 30 has therein a guide wire lumen 31 into which a guide wire is inserted. The proximal end of the inner shaft 30 is fixed to the guide wire port 23 of the outer shaft 20 to form a proximal end side guide wire port 32. A technician can exchange guide wires through the proximal end side guide wire port 32.

As described later, the distal end tip 40 is fixed to the distal end of the inner shaft 30. The distal end tip 40 is formed of flexible resin. The material is not particularly limited, and polyurethane, polyurethane elastomer, and the like can be used. Moreover, the distal end tip 40 has a distal end side guide wire port 41 on its distal end.

The reinforcing body 50 is attached on an inner periphery of the distal end of the proximal end outer shaft part 25. The reinforcing body 50 has a circular cross section, and is metal wire tapered toward the distal end. The material of the reinforcing body 50 is not particularly limited, and stainless steel (SUS304) or a superelastic alloy such as a Ni-Ti alloy can be used. The reinforcing body 50 passes the intermediate outer shaft part 24 and the guide wire port part 23 and extends to the distal end outer shaft part 22.

As illustrated in FIG. 3A, there are provided, at the proximal end 12 of the balloon 10, an outer projection 70 extending in an axis direction, an inner projection 72 extending in the axis direction, and a gap 71 formed between the outer projection 70 and the inner projection 72. As illustrated in FIG. 3B, there are provided, at the distal end 21 of the outer shaft 20, an outer projection 80 extending in the axis direction, an inner projection 82 extending in the axis direction, and a gap 81 formed between the outer projection 80 and the inner projection 82.

In the balloon catheter 1 of the embodiment, the inner projection 72 of the balloon 10 is disposed in the gap 81 of the outer shaft 20, and the outer projection 80 of the outer shaft 20 is disposed in the gap 71 of the balloon 10 (see FIG. 2). In other words, at a fixing part 14 between the proximal end 12 of the balloon 10 and the distal end 21 of the outer shaft 20, there are sequentially laminated in a diameter direction the inner projection 82 of the outer shaft 20, the inner projection 72 of the balloon 10, the outer projection 80 of the outer shaft 20, and the outer projection 70 of the balloon 10.

In this manner, the proximal end 12 of the balloon 10 and the distal end 21 of the outer shaft 20 are engaged with each other, whereby a fixing area between the balloon 10 and the outer shaft 20 can be expanded easily. Thus, it is possible to improve the fixing strength between the balloon 10 and the outer shaft 20 without increasing an outer diameter at the fixing part 14 between the balloon 10 and the outer shaft 20. As a result, it is possible to reduce a risk that the proximal end 12 of the balloon 12 is removed from the distal end 21 of the outer shaft 20 even when a technician imposes high pressure on the balloon 10.

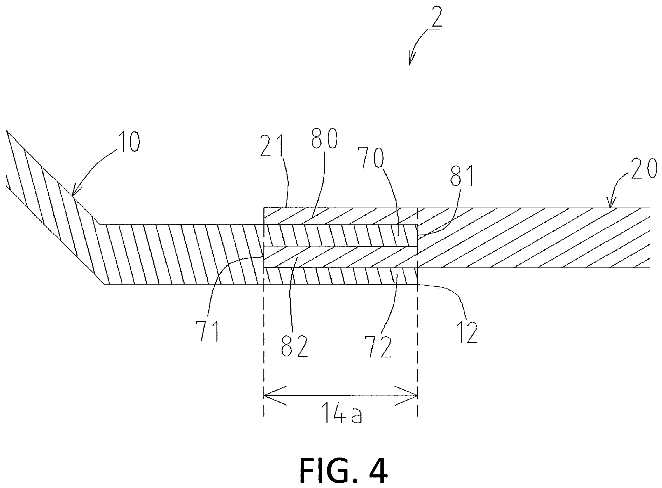

Next, a balloon catheter 2 of the second embodiment will be described with reference to FIG. 4. FIG. 4 is a modification of FIG. 2. Explaining only a difference from the balloon catheter 1, in the balloon catheter 2, the outer projection 70 of the balloon 10 is disposed in the gap 81 of the outer shaft 20, and the inner projection 82 of the outer shaft 20 is disposed in the gap 71 of the balloon 10 (see FIG. 4). In other words, at a fixing part 14a between the proximal end 12 of the balloon 10 and the distal end 21 of the outer shaft 20, there are sequentially laminated in the diameter direction the inner projection 72 of the balloon 10, the inner projection 82 of the outer shaft 20, the outer projection 70 of the balloon 10, and the outer projection 80 of the outer shaft 20.

The fixing part 14a of the balloon catheter 2 has a structure in which the proximal end 12 of the balloon 10 and the distal end 21 of the outer shaft 20 are engaged with each other, similarly to the fixing part 14 of the balloon catheter 1. Thus, a fixing area between the balloon 10 and the outer shaft 20 can be expanded easily. Thus, it is possible to improve the fixing strength between the balloon 10 and the outer shaft 20 without increasing an outer diameter at the fixing part 14a between the balloon 10 and the outer shaft 20. As a result, it is possible to reduce a risk that the proximal end 12 of the balloon 10 is removed from the distal end 21 of the outer shaft 20 even when a technician imposes a high pressure on the balloon 10.

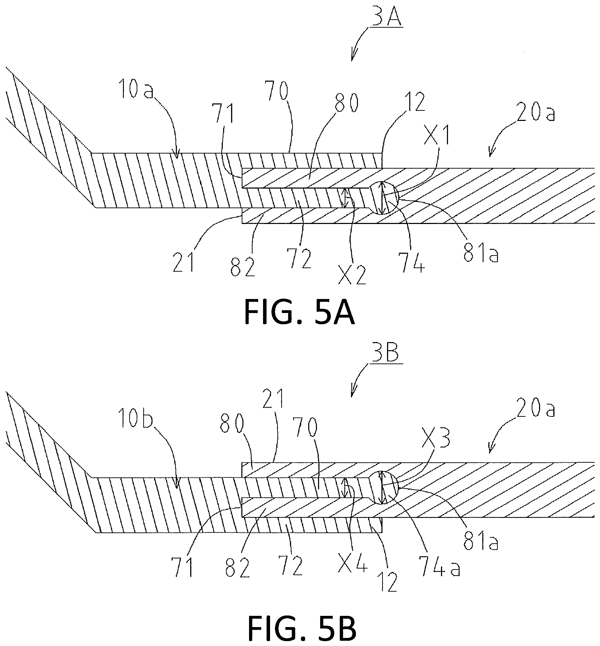

Next, balloon catheters 3A, 3B of the third embodiment will be described with reference to FIG. 5A and FIG. 5B. FIG. 5A is a modification of FIG. 2. FIG. 5B is a modification of FIG. 4. Explaining only a difference from the balloon catheter 1, in the balloon catheter 3A, a bulging portion 74 is provided at the proximal end of the inner projection 72 of a balloon 10a disposed in a gap 81a of an outer shaft 20a, as illustrated in FIG. 5A. The bulging portion 74 is formed of the same material as the inner projection 72, and is larger in the diameter direction than the inner projection 72. In other words, a thickness X1 of the bulging portion 74 is larger than a thickness X2 of the inner projection 72 (X1 > X2).

Similarly, explaining only a difference from the balloon catheter 2, in the balloon catheter 3B, a bulging portion 74a is provided at the proximal end of the outer projection 70 of a balloon 10b disposed in the gap 81a of the outer shaft 20a, as illustrated in FIG. 5B. The bulging portion 74a is formed of the same material as the outer projection 70, and is larger in the diameter direction than the outer projection 70. In other words, a thickness X3 of the bulging portion 74a is larger than a thickness X4 of the outer projection 70 (X3 > X4).

In this manner, the bulging portion 74, 74a provided at the proximal end of the inner projection 72 or the outer projection 70 is disposed in the gap 81a of the outer shaft 20a, whereby a fixing area between the balloons 10a, 10b and the outer shaft 20a can be further expanded, and the anchoring effect of the bulging portion 74, 74a relative to the gap 81a of the outer shaft 20a can further improve the fixing strength between the balloon 10a, 10b and the outer shaft 20a.

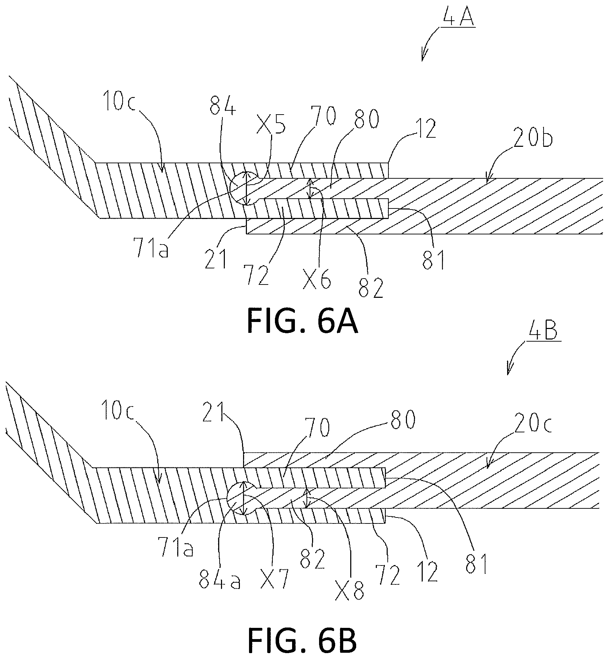

Next, balloon catheters 4A, 4B of the fourth embodiment will be described with reference to FIG. 6A and FIG. 6B. FIG. 6A is a modification of FIG. 2. FIG. 6B is a modification of FIG. 4. Explaining only a difference from the balloon catheter 1, in the balloon catheter 4A, a bulging portion 84 is provided at the distal end of the outer projection 80 of an outer shaft 20b disposed in a gap 71a of a balloon 10c, as illustrated in FIG. 6A. The bulging portion 84 is formed of the same material as the outer projection 80, and is larger in the diameter direction than the outer projection 80. In other words, a thickness X5 of the bulging portion 84 is larger than a thickness X6 of the outer projection 80 (X5 > X6).

Similarly, explaining only a difference from the balloon catheter 2, in the balloon catheter 4B, a bulging portion 84a is provided at the distal end of the inner projection 82 of an outer shaft 20c disposed in the gap 71a of the balloon 10c, as illustrated in FIG. 6B. The bulging portion 84a is formed of the same material as the inner projection 82, and is larger in the diameter direction than the inner projection 82. In other words, a thickness X7 of the bulging portion 84a is larger than a thickness X8 of the inner projection 82 (X7 > X8).

In this manner, the bulging portion 84, 84a provided at the distal end of the outer projection 80 or the inner projection 82 is disposed in the gap 71a of the balloon 10c, whereby the fixing area between the balloon 10c and the outer shaft 20b, 20c can be further expanded, and the anchoring effect of the bulging portion 84, 84a relative to the gap 71a of the balloon 10c can further improve the fixing strength between the balloon 10c and the outer shaft 20b, 20c.

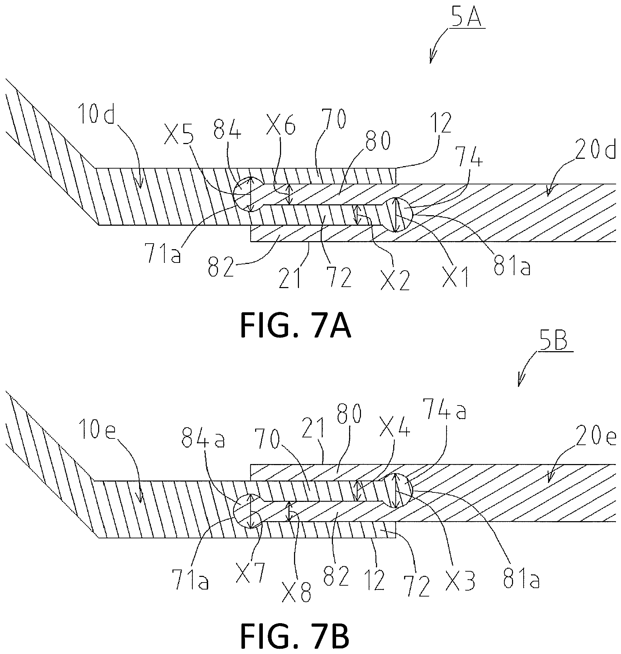

Next, balloon catheters 5A, 5B of the fifth embodiment will be described with reference to FIG. 7A and FIG. 7B. FIG. 7A is a modification of FIG. 6A. FIG. 7B is a modification of FIG. 6B. Explaining only a difference from the balloon catheters 4A, 4B, in the balloon catheter 5A, the bulging portion 74 is provided, in addition to the bulging portion 84, at the proximal end of the inner projection 72 of a balloon 10d disposed in the gap 81a of an outer shaft 20d, as illustrated in FIG. 7A. Note that the bulging portion 74 is same as the balloon catheter 3A described in FIG. 5A, and thus the explanation thereof is omitted.

Similarly, explaining only a difference from the balloon catheter 4B, in the balloon catheter 5B, the bulging portion 74a is provided, in addition to the bulging portion 84a, at the proximal end of the outer projection 70 of a balloon 10e disposed in the gap 81a of an outer shaft 20e, as illustrated in FIG. 7B. Note that the bulging portion 74a is same as the balloon catheter 3B described in FIG. 5B, and thus the explanation thereof is omitted.

In this manner, the bulging portion 84, 84a provided at the distal end of the outer projection 80 or the inner projection 82 is disposed in the gap 71a of the balloon 10d, 10e, and the bulging portion 74, 74a provided at the proximal end of the inner projection 72 or the outer projection 70 is disposed in the gap 81a of the outer shaft 20d, 20e, whereby the fixing area between the balloon 10d, 10e and the outer shafts 20d, 20e can be further expanded. In addition, the anchoring effect of the bulging portions 84, 84a relative to the gap 71a of the balloon 10d, 10e, and the anchoring effect of the bulging portion 74, 74a relative to the gap 81a of the outer shaft 20d, 20e can further improve the fixing strength between the balloon 10d, 10e and the outer shaft 20d, 20e.

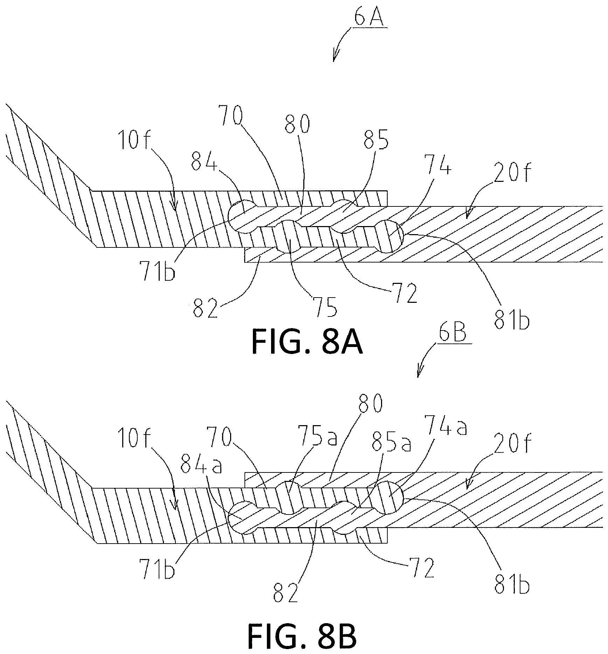

The balloon catheter 5A to 6B includes one bulging portion 74 at the proximal end of the inner projection 72 of the balloon 10a, 10d, one bulging portion 74a at the proximal end of the outer projection 70 of the balloon 10b, 10e, one bulging portion 84 at the distal end of the outer projection 80 of the outer shaft 20b, 20d, and one bulging portion 84a at the distal end of the inner projection 82 of the outer shaft 20c, 20e. The number and the position of the bulging portions 74, 74a, 84, 84a are not particularly limited. For example, in a balloon catheter 6A of the sixth embodiment illustrated in FIG. 8A, two bulging portions 84, 85 provided at the distal end and the middle of the outer projection 80 of an outer shaft 20f may be disposed in a gap 71b of a balloon 10f, and two bulging portions 74, 75 provided at the middle and the proximal end of the inner projection 72 of the balloon 10f may be disposed in a gap 81b of the outer shaft 20f. Moreover, in the balloon catheter 6B of the sixth embodiment illustrated in FIG. 8B, two bulging portions 84a, 85a provided at the distal end and the middle of the inner projection 82 of the outer shaft 20f may be disposed in the gap 71b of the balloon 10f, and two bulging portions 74a, 75a provided at the middle and the proximal end of the outer projection 70 of the balloon 10f may be disposed in the gap 81b of the outer shaft 20f.

Note that the form of the bulging portions 74, 74a, 75, 75a, 84, 84a, 85, 85a is not limited to the form illustrated in FIG. 5A to FIG. 8B as long as the anchoring effect can be obtained.

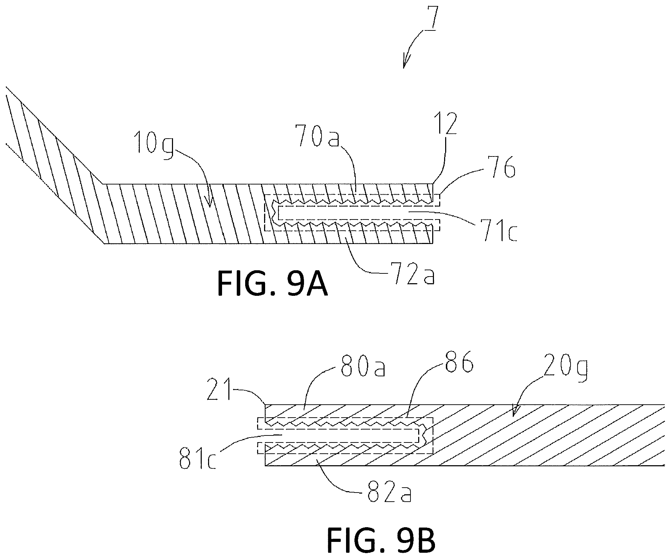

Instead of the above-described bulging portions 74, 74a, 75, 75a, 84, 84a, 85, 85a, an uneven inner peripheral surface 76 may be provided in the gap 71c of the balloon 10g, and an uneven inner peripheral surface 86 may be provided in the gap 81c of the outer shaft 20g, as illustrated in a balloon catheter 7 of the seventh embodiment in FIGS. 9A, 9B.

To be more specific, there are provided, at the proximal end 12 of a balloon 10g, an outer projection 70a extending in the axis direction, an inner projection 72a extending in the axis direction, and a gap 71c that is formed between the outer projection 70a and the inner projection 72a and has the uneven inner peripheral surface 76, as illustrated in FIG. 9A. Moreover, as illustrated in FIG. 9B, there are provided, at the distal end 21 of an outer shaft 20g, an outer projection 80a extending in the axis direction, an inner projection 82a extending in the axis direction, and a gap 81c that is formed between the outer projection 80a and the inner projection 82a and has the uneven inner peripheral surface 86. Similarly to FIGS. 3A, 3B, and FIGS. 9A, 9B illustrated, for facilitating understanding, the state where the balloon 10g and the outer shaft 20g are separated from each other.

In such a manner, the inner peripheral surface 76 is provided in the gap 71c of the balloon 10g, and the inner peripheral surface 86 is provided in the gap 81c of the outer shaft 20g. Then, the inner peripheral surface 76 and the inner peripheral surface 86 are fixed to each other, whereby the fixing area between the balloon 10g and the outer shaft 20g can be expanded. As a result, it is possible to improve the fixing strength between the balloon 10g and the outer shaft 20g.

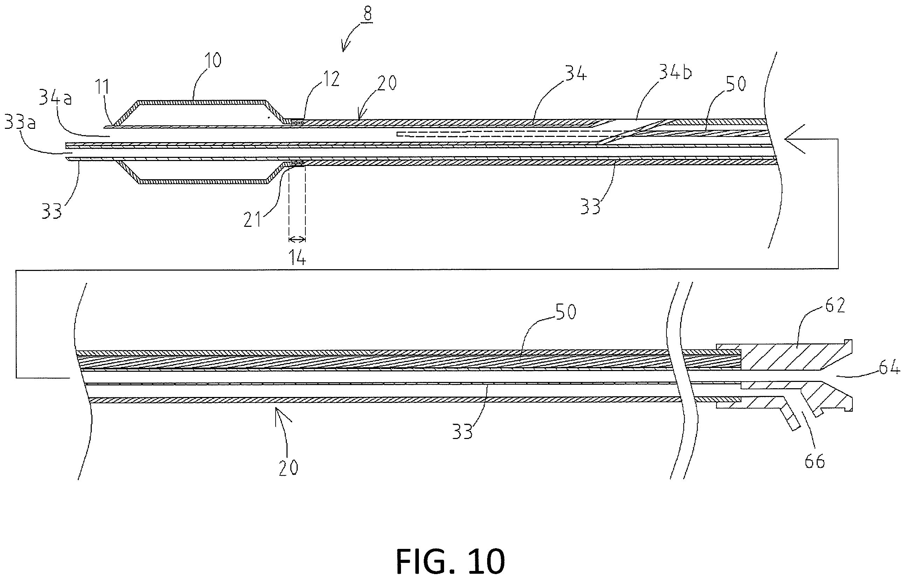

Next, a balloon catheter 8 of the eighth embodiment will be described with reference to FIG. 10. In FIG. 10, the left side of the drawing is a distal end side to be inserted in a body (far side), while the right side is a proximal end side to be operated by a technician such as a physician (near side, rear end side). In the balloon catheter 8, the same symbols as in the balloon catheters 1 to 7 described in the first to the seventh embodiments represent the same members unless otherwise specified.

The balloon catheter 8 is for example, a fixing balloon catheter that facilitates operation of inserted medical equipment such as a guide wire by expanding a balloon to a blood vessel wall or a digestive organ wall and fixing the balloon. As illustrated in FIG. 10, the balloon catheter 8 mainly includes the balloon 10, the outer shaft 20, a first inner shaft 34, a second inner shaft 33, the reinforcing body 50, and a connector 62.

In the outer shaft 20, the second inner shaft 33 is inserted in the whole length of the balloon catheter 8. A guide wire can be inserted into the second inner shaft 33. In order to facilitate insertion of the guide wire, the connector 62 is connected to the proximal end of the outer shaft 20 and the proximal end of the second inner shaft 33. A first distal end port 33a is provided at the distal end of the second inner shaft 33, and a first insertion port 64 is provided at the proximal end of the second inner shaft 33 through the connector 62.

In the outer shaft 20, the first inner shaft 34 is inserted, in parallel to the second inner shaft 33, in the middle to the distal end of the balloon catheter 8. Similarly to the second inner shaft 33, the guide wire can be inserted also into the first inner shaft 34. A second distal end port 34a is provided at the distal end of the first inner shaft 34, and a second insertion port 34b is provided at the proximal end of the first inner shaft 34.

The outer shaft 20, the first inner shaft 34, and the second inner shaft 33 are formed of thermoplastics resin. Resins, for example; polyamide, polyamide elastomer, polyolefin, polyester, polyester elastomer, and nylon, can be used.

The second inner shaft 33 extends over the whole length of the balloon catheter 8. Thus, there is a demerit that it is difficult for the technician to change the guide wire inserted in the second inner shaft 33 because the second inner shaft 33 is long. By contrast, there is a merit that with the guide wire inserted in the second inner shaft 33, the technician can easily push the balloon catheter 8 into the distal end direction because the guide wire improves rigidity of the balloon catheter 8. Moreover, the first inner shaft 34 extends only from the middle to the distal end of the balloon catheter 8. Thus, there is a merit that the technician can easily change the guide wire inserted in the first inner shaft 34. By contrast, there is a demerit that even with the guide wire inserted in the first inner shaft 34, the rigidity of the balloon catheter 8 improves only at the distal end side, and the balloon catheter 8 is broken around the second insertion port 34b of the first inner shaft 34 where the rigidity changes suddenly when the technician pushes the balloon catheter 8 into the distal end direction. The balloon catheter 8 includes both the first inner shaft 34 and the second inner shaft 33. Thus, for example, the technician can rapidly change the second guide wire inserted in the first inner shaft 34 while the first guide wire is inserted in the second inner shaft 33, and the balloon catheter 8 can be easily pushed into the distal end direction.

As illustrated in FIG. 10, the balloon 10 fixed to a blood vessel wall or a digestive organ wall is formed of a resin member, and includes the distal end attachment part 11 at the distal end of the balloon 10, and the proximal end attachment part 12 at the proximal end of the balloon 10. The distal end attachment part 11 is fixed to the first inner shaft 34 and the second inner shaft 33, while the proximal end attachment part 12 is fixed to the distal end 21 of the outer shaft 20.

In order to expand the balloon 10, an expanded lumen for supplying liquid such as a contrast medium or physiological saline is provided between the outer shaft 20, and the first inner shaft 34 and the second inner shaft 33, although it is not illustrated. A liquid supply port 66 to which an indeflator (not illustrated) can be attached is provided in the connector 62. The liquid such as a contrast medium or physiological saline supplied from the liquid supply port 66 passes through the expanded lumen to the balloon 10, then expanding the balloon 10.

In the balloon catheter 8, the metal reinforcing body 50 extending in an axis direction is inserted between the outer shaft 20 and the second inner shaft 33. The distal end of the reinforcing body 50 extends more to the distal end side than the second insertion port 34b of the first inner shaft 34.

Similarly to the balloon catheters 1 to 7, in the balloon catheter 8, the proximal end 12 of the balloon 10 and the distal end 21 of the outer shaft 20 are engaged with each other, whereby a fixing area between the balloon 10 and the outer shaft 20 can be expanded easily. Thus, it is possible to improve the fixing strength between the balloon 10 and the outer shaft 20 without increasing an outer diameter at the fixing part 14 between the balloon 10 and the outer shaft 20. As a result, even when a technician imposes a high pressure on the balloon 10, it is possible to reduce a risk that the proximal end 12 of the balloon 10 is removed from the distal end 21 of the outer shaft 20.

### Reference Signs List

- 1 to 8: balloon catheter
- 10 to 10g: balloon
- 11: distal end of balloon
- 12: proximal end of balloon
- 14, 14a: fixing part
- 20 to 20g: outer shaft
- 30: inner shaft
- 33: second inner shaft
- 34: first inner shaft
- 40: distal end tip
- 50: reinforcing body
- 60, 62: connector
- 70, 70a: outer projection of balloon
- 71, 71a, 71b, 71c: gap of balloon
- 72, 72a: inner projection of balloon
- 74, 74a, 75, 75a: bulging portion
- 76: inner peripheral surface
- 80, 80a: outer projection of outer shaft
- 81, 81a, 81b, 81c: gap of outer shaft
- 82, 82a: inner projection of outer shaft
- 84, 84a, 85, 85a: bulging portion
- 86: inner peripheral surface

## Claims

1. A balloon catheter (1, 2, 3A, 3B, 4A, 4B, 5A, 5B, 6A, 6B, 7, 8) comprising:
a balloon (10, 10a, 10b, 10c, 10d, 10e, 10f, 10g) ; and
an outer shaft (20, 20a, 20b, 20c, 20d, 20e, 20f, 20g) fixed to a proximal end (12) of the balloon (10, 10a, 10b, 10c, 10d, 10e, 10f, 10g), **characterised in that**:
each of the proximal end (12) of the balloon (10, 10a, 10b, 10c, 10d, 10e, 10f, 10g) and a distal end (21) of the outer shaft (20, 20a, 20b, 20c, 20d, 20e, 20f, 20g) has an inner projection (72, 72a, 82, 82a) extending in an axis direction, an outer projection (70, 70a, 80, 80a) extending in the axis direction, and a gap (71, 71a, 71b, 71c, 81, 81a, 81b, 81c) formed between the inner projection (72, 72a, 82, 82a) and the outer projection (70, 70a, 80, 80a); and
i) the inner projection (72, 72a) of the balloon (10, 10a, 10c, 10d, 10f, 10g) is disposed in the gap (81, 81a, 81b, 81c) of the outer shaft (20, 20a, 20b, 20d, 20f, 20g) and the outer projection (80, 80a) of the outer shaft (20, 20a, 20b, 20d, 20f, 20g) is disposed in the gap (71, 71a, 71c) of the balloon (10, 10a, 10c, 10d, 10f, 10g), or
ii) the outer projection (70, 70a) of the balloon (10, 10b, 10c, 10e, 10f, 10g) is disposed in the gap (81, 81a, 81b, 81c) of the outer shaft (20, 20a, 20b, 20e, 20f, 20g) and the inner projection (82, 82a) of the outer shaft (20, 20a, 20c, 20e, 20f, 20g) is disposed in the gap (71, 71a, 71b, 71c) of the balloon (10, 10b, 10c, 10e, 10f, 10g).

2. The balloon catheter (3A, 3B, 5A, 5B, 6A, 6B,) according to claim 1, wherein a bulging portion (74, 74a, 75, 75a) is provided at the inner projection (72) of the balloon (10a, 10d, 10f) or the outer projection (70) of the balloon (10b, 10e, 10f) that is disposed in the gap (81a, 81b) of the outer shaft (20a, 20d, 20e, 20f).

3. The balloon catheter (4A, 4B, 5A, 5B, 6A, 6B) according to claim 1 or claim 2, wherein a bulging portion (84, 84a, 85, 85a) is provided at the inner projection (82) of the outer shaft (20c, 20e, 20f) or the outer projection (84) of the outer shaft (20b, 20d, 20f) that is disposed in the gap (71a, 71b) of the balloon (10c, 10d, 10e, 10f).

## Patentansprüche

1. Ballonkatheter (1, 2, 3A, 3B, 4A, 4B, 5A, 5B, 6A, 6B, 7, 8) mit:
einem Ballon (10, 10a, 10b, 10c, 10d, 10e, 10f, 10g); und
einem äußeren Schaft (20, 20a, 20b, 20c, 20d, 20e, 20f, 20g), der an einem proximalen Ende (12) des Ballons (10, 10a, 10b, 10c, 10d, 10e, 10f, 10g) befestigt ist, **dadurch gekennzeichnet, dass**:
das proximale Ende (12) des Ballons (10, 10a, 10b, 10c, 10d, 10e, 10f, 10g) und ein distales Ende (21) des äußeren Schafts (20, 20a, 20b, 20c, 20d, 20e, 20f, 20g) jeweils einen inneren Vorsprung (72, 72a, 82, 82a), der sich in axialer Richtung erstreckt, einen äußeren Vorsprung (70, 70a, 80, 80a), der sich in der axialen Richtung erstreckt, und einen Spalt (71, 71a, 71b, 71c, 81, 81a, 81b, 81c) aufweisen, der zwischen dem inneren Vorsprung (72, 72a, 82, 82a) und dem äußeren Vorsprung (70, 70a, 80, 80a) ausgebildet ist; und
i) der innere Vorsprung (72, 72a) des Ballons (10, 10a, 10c, 10d, 10f, 10g) in dem Spalt (81, 81a, 81b, 81c) des äußeren Schafts (20, 20a, 20b, 20d, 20f, 20g) und der äußere Vorsprung (80, 80a) des äußeren Schafts (20, 20a, 20b, 20d, 20f, 20g) in dem Spalt (71, 71a, 71c) des Ballons (10, 10a, 10c, 10d, 10f, 10g) angeordnet ist, oder
ii) der äußere Vorsprung (70, 70a) des Ballons (10, 10b, 10c, 10e, 10f, 10g) in dem Spalt (81, 81a, 81b, 81c) des äußeren Schafts (20, 20a, 20b, 20e, 20f, 20g) und der innere Vorsprung (82, 82a) des äußeren Schafts (20, 20a, 20c, 20e, 20f, 20g) in dem Spalt (71, 71a, 71b, 71c) des Ballons (10, 10b, 10c, 10e, 10f, 10g) angeordnet ist.

2. Ballonkatheter (3A, 3B, 5A, 5B, 6A, 6B,) nach Anspruch 1, wobei an dem inneren Vorsprung (72) des Ballons (10a, 10d, 10f) oder dem äußeren Vorsprung (70) des Ballons (10b, 10e, 10f), der in dem Spalt (81a, 81b) des äußeren Schafts (20a, 20d, 20e, 20f) angeordnet ist, eine Ausbauchung (74, 74a, 75, 75a)vorgesehen ist.

3. Ballonkatheter (4A, 4B, 5A, 5B, 6A, 6B) nach Anspruch 1 oder Anspruch 2, wobei eine an dem inneren Vorsprung (82) des äußeren Schafts (20c, 20e, 20f) oder dem äußeren Vorsprung (84) des äußeren Schafts (20b, 20d, 20f), der in dem Spalt (71a, 71b) des Ballons (10c, 10d, 10e, 10f) angeordnet ist, eine Ausbauchung (84, 84a, 85, 85a) vorgesehen ist.

## Revendications

1. Cathéter à ballonnet (1, 2, 3A, 3B, 4A, 4B, 5A, 5B, 6A, 6B, 7, 8) comprenant :
un ballonnet (10, 10a, 10b, 10c, 10d, 10e, 10f, 10g) ; et
une tige externe (20, 20a, 20b, 20c, 20d, 20e, 20f, 20g) fixée à une extrémité proximale (12) du ballonnet (10, 10a, 10b, 10c, 10d, 10e, 10f, 10g), **caractérisé en ce que**
chacune de l'extrémité proximale (12) du ballonnet (10, 10a, 10b, 10c, 10d, 10e, 10f, 10g) et d'une extrémité distale (21) de la tige externe (20, 20a, 20b, 20c, 20d, 20e, 20f, 20g) a une saillie interne (72, 72a, 82, 82a) s'étendant dans une direction d'axe, une saillie externe (70, 70a, 80, 80a) s'étendant dans la direction d'axe, et un espace (71, 71a, 71b, 71c, 81, 81a, 81b, 81c) formé entre la saillie interne (72, 72a, 82, 82a) et la saillie externe (70, 70a, 80, 80a) ; et
i) la saillie interne (72, 72a) du ballonnet (10, 10a, 10c, 10d, 10f, 10g) est disposée dans l'espace (81, 81a, 81b, 81c) de la tige externe (20, 20a, 20b, 20d, 20f, 20g) et la saillie externe (80, 80a) de la tige externe (20, 20a, 20b, 20d, 20f, 20g) est disposée dans l'espace (71, 71a, 71c) du ballonnet (10, 10a, 10c, 10d, 10f, 10g), ou
ii) la saillie externe (70, 70a) du ballonnet (10, 10b, 10c, 10e, 10f, 10g) est disposée dans l'espace (81, 81a, 81b, 81c) de la tige externe (20, 20a, 20b, 20e, 20f, 20g) et la saillie interne (82, 82a) de la tige externe (20, 20a, 20c, 20e, 20f, 20g) est disposée dans l'espace (71, 71a, 71b, 71c) du ballonnet (10, 10b, 10c, 10e, 10f, 10g)

2. Cathéter à ballonnet (3A, 3B, 5A, 5B, 6A, 6B) selon la revendication 1, dans lequel une partie de renflement (74, 74a, 75, 75a) est prévue au niveau de la saillie interne (72) du ballonnet (10a, 10d, 10f) ou de la saillie externe (70) du ballonnet (10b, 10e, 10f) qui est disposée dans l'espace (81a, 81b) de la tige externe (20a, 20d, 20e, 20f).

3. Cathéter à ballonnet (4A, 4B, 5A, 5B, 6A, 6B) selon la revendication 1 ou la revendication 2, dans lequel une partie de renflement (84, 84a, 85, 85a) est prévue au niveau de la saillie interne (82) de la tige externe (20c, 20e, 20f) ou de la saillie externe (84) de la tige externe (20b, 20d, 20f) qui est disposée dans l'espace (71a, 71b) du ballonnet (10c, 10d, 10e, 10f).
